# EUROPEAN PATENT SPECIFICATION

(11) **EP 2 925 287 B1**
(45) Date of publication and mention of the grant of the patent: **14.09.2016**
(21) Application number: 13791981.7
(22) Date of filing: 12.11.2013
(51) Int. Cl.: A61K 8/49, A61Q 5/00, A61K 8/81, A61K 8/23, A61Q 5/02, A61Q 5/12, A61K 8/891, A61K 8/892

(54) **HAIR CARE COMPOSITION**
HAARPFLEGEZUSAMMENSETZUNG
COMPOSITION DE SOIN CAPILLAIRE

(30) Priority: 29.11.2012 WO PCT/CN2012/085557; 14.01.2013 EP 13151135
(43) Date of publication of application: 07.10.2015
(73) Proprietor: Unilever PLC, London, Greater London EC4Y 0DY (GB); Unilever N.V., 3013 AL Rotterdam (NL)
(72) Inventor: HENTRICH, Doreen, 14471 Potsdam (DE); JARVIS, Adam, Peter, Wirral Merseyside CH63 3JW (GB); KITA-TOKARCZYK, Katarzyna, Elzbieta, 65812 Bad Soden am Taunus (DE); LIMER, Adam, John, Shanghai 200335 (CN); ROBSON, Scott, Wirral Merseyside CH63 3JW (GB)
(74) Representative: James, Helen Sarah
(86) International application number: PCT/EP2013/073607
(87) International publication number: WO 2014/082851

(56) References cited:
- FR-A1- 2 920 975
- US-A- 4 892 916

## Description

This invention relates to an aqueous cleansing composition comprising silicone conditioning agents.

Compositions comprising anionic cleansing surfactants are formulated to provide a microstructure of entangled elongated or rod-like micelles. However, the native yield stress of such compositions is very low so there is a need for an additional suspending agent if it is desired to include colloidal benefit agents such as oily emulsion droplets such as silicones or particulates such as coated mica or insoluble anti-dandruff agents. Traditionally, suspension in shampoo formulations has been achieved either by adding associative thickeners (polymers that build structure by cross-linking the surfactant micelles) or space-filling particles.

The former class of suspending agent is typified by the Hydrophically-modified Alkali Swellable Emulsion (HASE) polymers such as Aculyn 28 ex Rohm & Haas or Carbopol Aqua SF1 ex-Lubrizol. These polymers comprise a polyacrylate backbone, whose solubility is controlled by pH, and hydrophobic pendant groups that associate with surfactant micelles to form a network. Such networks are typically optically transparent and hence offer a combination of clarity and suspending power. However it has been found that, due to the strength of the network thus formed, it may be difficult to break the product down during use. This reduces the efficiency with which the shampoo formulation can release and deposit colloidal benefit agents such as silicone oil droplets.

Thus there remains the need for alternative suspending agents that can suspend materials such as silicone conditioning materials, do not adversely affect the appearance of the product and do not inhibit the deposition of the suspended material from the product.

The present invention relates to a composition comprising a suspending agent that is weight efficient so less suspending agent is required to suspend a material within the composition. The fact that less suspending agent is required means that deposition of the material is not adversely effected compared with a conventional composition which requires higher levels of suspending agents.

The present invention relates to an aqueous composition comprising:
i) at least 0.5 wt. % of the total composition of a cleansing surfactant
ii) at least 0.01 wt. % of the total composition of a silicone conditioning agent;
iii) at least 0.05 wt. % of the addition polymerization product of:
   a. from 0.1 to 5 wt. % of a first unsaturated monomer A of an ethylenically unsaturated cis diacid of formula (I):

      HOOC-CR₅=CR₆-COOH (I)

      or an unsaturated cyclic anhydride precursor of such an ethylenically unsaturated diacid, the anhydride having formula (II) 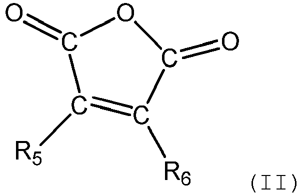 where R₅ and R₆ are individually selected from H, C₁-C₃ alkyl, phenyl, chlorine and bromine;
   b. from 1 to 60 wt. % of a second ethylenically unsaturated monomer B selected from acrylic acid, methacrylic acid and combinations thereof,
   c. from 30 to 75 wt. % a (meth)acrylate monomer C selected from C₁ to C₈ alkyl esters of (meth)acrylic acid and C₁ to C₈ alkyl esters of methacrylic acid and combinations thereof,
   d. from 1 to 25 wt. % of an associative monomer D of the formula III: 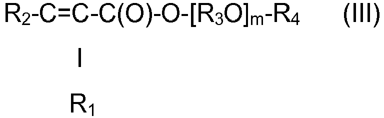
      where R₁ and R₂ are each independently selected from H, and C₁₋₃ alkyl; R₃ is C₂-C₄ and mixtures thereof, preferably C₂;
      m, the average number of alkoxy units R₃O, is from 6 to 40;
      R₄ is alkyl or alkylaryl where the alkyl part is linear or branched; and the total number of carbons is from 6 to 40.
   e. from 0 to 1.0 wt. % of a cross linking monomer E for introducing branching and controlling molecular weight, the cross linking monomer comprising polyfunctional units carrying multiple reactive functionalization groups selected from the group consisting of vinyl, allylic and functional mixtures thereof.

The invention also relates to a method of treating the hair with the above mentioned composition.

### Detailed Description of the Invention

### Copolymer

The copolymers of the invention are based on a combination of monomers that include an ethylenically unsaturated polyacidic monomer such as a maleic derivative, a monoacid ethylenically unsaturated monomer such as an acrylic or methacrylic acid, an acrylate ester monomer an associative monomer and optionally a crosslinker.

Preferably the level of copolymer in the invention is from 0.05 to 1 wt. % of the total composition; more preferably from 0.1 to 0.7 wt. %, most preferably from 0.2 to 0.5 wt. %.

Preferably the copolymer is swollen, more preferably fully swollen in an aqueous solution. The term swollen means an increase in the structured volume of a solution associated with the uptake of a liquid. The term fully swollen means that no further liquid can be uptaken (and hence no increase in viscosity occurs). To obtain such a fully swollen polymer it is preferable if the polymer is dissolved in water and the pH increased until no further change in viscosity occurs. The fully swollen polymer can then be added to the formulation or the formulation is built around the fully swollen polymer. It is preferred if the polymer is swollen before addition of the surfactant.

It is preferable if ranges of the monomeric units in the polymer are as follows:
Monomer A ranges from 0.2 to 1 % by weight of the copolymer;
Monomer B ranges from 25 to 50 % by weight of the copolymer;
Monomer C ranges from 30 to 65 % by weight of the copolymer; and
Monomer D ranges from 2 to 12 % by weight of the copolymer.

In the context of the present invention the weight % of monomer within the copolymer relates to the non-neutralized monomer (not it salt form).

Preferably the weight ratio of monomer A to monomer D is from 1:3 to 1:30; more preferably from 1:5 to 1:25; most preferably from 1:7 to 1:22. These weight ratios are particularly advantageous with the preferred monomers of A and D listed below.

Preferably the copolymer includes neutralized and partially neutralized (non-protonated) forms.

### Monomer A

Monomer A is an ethylenically unsaturated diacid, preferably cis diacid of formula (I):

HOOC-CR₅=CR₆-COOH (I)

or an unsaturated cyclic anhydride precursor of such an ethylenically unsaturated diacid, the anhydride having formula (II) 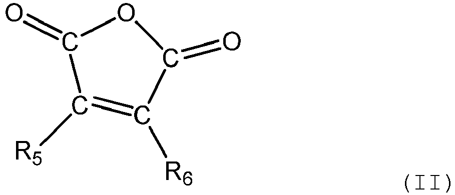 where R₅ and R₆ are individually selected from H, C₁-C₃ alkyl, phenyl, chlorine and bromine;

Preferably the copolymer will include a first ethylenically unsaturated monomer which may be maleic, fumaric, itaconic and citraconic acids and anhydrides thereof as well as combinations of these. Most preferred are maleic derivatives. Accordingly, the most preferred species are maleic acid, maleic anhydride or a combination thereof. Preferably maleic acid can be generated from maleic anhydride as starting material and hydrolyzing this to the di acid in the emulsion polymerization.

While both fumaric and itaconic acid are contemplated for use herein, in one or more embodiments, it is preferred the itaconic acid and, in further embodiments, both itaconic and fumaric acid, are absent from the subject copolymers or are individually present in only minor amounts, i.e., less than 0.05 wt. %, preferably less than 0.005 wt. %, based on the total weight of monomer. While both fumaric and itaconic acid are contemplated for use herein, in one or more embodiments, it is preferred the itaconic acid and, in further embodiments, both itaconic and fumaric acid, are absent from the subject copolymers or are individually present in only minor amounts, i.e., less than 0.05 wt. %, preferably less than 0.005 wt. %, based on the total weight of monomer.

The first ethylenically unsaturated acid monomer may be employed in amounts of from 0.1 to 5 wt. %, preferably from 0.2 to 4 wt. %, more preferably from 0.2 to 1 wt. %, and, in one or more embodiments, from 0.3 to 0.6 wt. %, based on the total weight of monomer. In one or more embodiments of particular interest, maleic acid accounts for at least 50 % by weight, more preferably at least 80 % by weight, even more preferably, at least 95 % by weight, based on the total weight of the first ethylenically unsaturated acid monomer.

### Monomer B

The second ethylenically unsaturated acid monomer B is selected from acrylic acid, methacrylic acid and combinations thereof.

The second ethylenically unsaturated acid monomer may be employed in amounts of from 15 to 60 wt. %, preferably from 20 to 55 wt. %, more preferably from 25 to 50 wt. %, based on total monomer. In one or more embodiments of interest the amount of second ethylenically unsaturated acid monomer is from 40 to 50 wt. % based on total monomer; in one or more other embodiments of interest, the amount of second ethylenically unsaturated acid monomer is from 20 to 40 wt. % based on total monomer. In one or more embodiments of particular interest methacrylic acid accounts for at least 50 % by weight, more preferably at least 70 % by weight, even more preferably, at least 90 % by weight, of the total weight of the second ethylenically unsaturated acid monomer.

### Monomer C

The (meth)acrylate monomer C may be selected from C₁ to C₈ alkyl esters of (meth)acrylic acid and C₁ to C₈ alkyl esters of methacrylic acid and combinations thereof, with C₁ to C₄ alkyl esters of such acids, being particularly preferred. Preferred ester monomers are ethyl acrylate, methyl acrylate, ethyl methacrylate, methyl methacrylate, butyl acrylate, and butyl methacrylate, ethyl acrylates is particularly preferred. In one or more embodiments of particular interest, C₁ to C₄ alkyl esters of acrylic acid, preferably ethyl acrylate, account, for at least 50 % by weight, more preferably at least 70 % by weight, even more preferably, at least 90 % by weight of the (meth)acrylate monomer.

### Monomer D

D is preferably an associative monomer of formula (I) 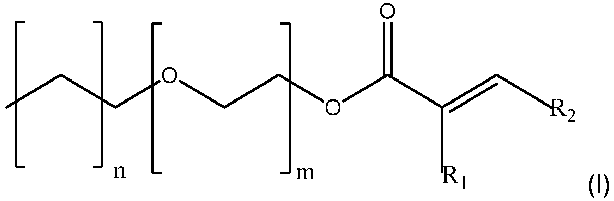 in which each R₁ and R₂ are independently selected from H, C₁ to C₃ alkyl

Preferably R₁ is a methyl group and R₂ is H.

n ranges from 6 to 40 and m ranges from 6 to 40, more preferably n ranges from 10 to 30 and m ranges 15 to 35 most preferably n ranges from 12 to 22 and m ranges from 20 to 30. It is preferable if m is greater or equal to n.

The associative monomer may be employed in amounts of from 1 to 25 wt. %, preferably from 2 to 20 wt. %, and more preferably from 2 to12 wt. %, based on total monomer. In one or more embodiments of particular interest the amount of associate monomer employed is from 5 to 10 wt. %, based on total monomer.

### Monomer E

One or more cross linking monomers E may be present in the copolymer for purposes of introducing branching and controlling molecular weight. These monomers will be polyunsaturated. Illustrative but not limiting examples are divinyl benzene, divinyl naphthalene, trivinyl benzene, triallyl pentaerythritol, diallyl pentaerythritol, diallyl sucrose, octaallyl sucrose, trimethylol propane diallyl ether, 1,6-hexanediol di(meth) acrylate, tetramethylene tri(meth) acrylate, trimethylol propane tri(meth)acrylate, polyethoxylated glycol di(meth) acrylate, alkylene bisacrylamides, bisphenol A polyethyoxylated dimethacrylate, trimethylolpropane polyethoxylated trimethacrylate and similar materials. Preferred for the present invention is bisphenol A polyethoxylated glycol diacrylate, diallyl pentaerythritol and trimethylolpropane triacrylate.

Amounts of the cross linking monomer may range from 0 to 1 wt. %, preferably from about 0 to 3 %, more preferably from about 0 to 2 % based on total monomer, optimally the copolymer is free from cross linking polymer.

In some embodiments, one or more the acid groups of the starting acid monomers may be neutralized to salt form. Typical salt counter-ions to the acid groups are alkali metals, especially sodium and potassium, and ammonium and triethanolammonium cations.

### Other Ingredients

Compositions in accordance with the invention are preferably formulated as compositions for washing the hair and subsequent rinsing.

### Cleansing Surfactant

Compositions of the invention comprise one or more cleansing surfactants which are cosmetically acceptable and suitable for topical application to the hair. Further surfactants may be present as emulsifiers.

Suitable cleansing surfactants are selected from anionic, amphoteric and zwitterionic surfactants, and mixtures thereof. The cleansing surfactant may be the same surfactant as the emulsifier, or may be different.

Compositions of the invention comprise anionic surfactant. Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae R₂OSO₃M and R₁O(C₂H₄O)ₓSO₃M, wherein R₂ is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably R₂ has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Preferably the level of alkyl ether sulphate is from 0.5 wt. % to 25 wt. % of the total composition, more preferably from 3 wt. % to 18 wt. %, most preferably from 6 wt. % to 15 wt. % of the total composition.

The total amount of anionic cleansing surfactant in compositions of the invention generally ranges from 0.5 wt. % to 45 wt. %, more preferably from 1.5 wt. % to 20 wt. %.

Compositions of the invention may comprise fatty acyl isethionate, if present preferably at a level of from 1 to 10 wt. %, more preferably from 2 to 8 wt. %, most preferably from 2.5 to 7.5 wt. %.

A preferred fatty acyl isethionate product comprises fatty acyl isethionate surfactant at a level of from 40 to 80 wt. % of the product, as well as free fatty acid and/or fatty acid salt at a level of from 15 to 50 %.

Preferably, greater than 20 wt. % and less than 45 wt. %, more preferably greater than 25 wt. % and less than 45 wt. % of the fatty acyl isethionate are of chain length greater than or equal to C₁₆; and greater than 50 wt. %, preferably greater than 60 wt. % of the free fatty acid/soap is of chain length C₁₆ to C₂₀.

In addition, the product may contain isethionates salts which are present typically at levels less than 5 wt. %, and traces (less than 2 wt. %) of other impurities.

Preferably, a mixture of aliphatic fatty acids is used for the preparation of commercial fatty acyl isethionates surfactants. The resulting fatty acyl isethionate surfactants (e.g., resulting from reaction of alkali metal isethionate and aliphatic fatty acid) preferably should have more than 20 wt. %, preferably more than 25 wt. %, but no more than 45 wt. %, preferably 35 % (on basis of fatty acyl isethionates reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

Examples of commercial fatty acyl isethionate products that are particularly useful in the subject invention are DEFI flakes and Dove^{®} cleansing bar noodles produced by Unilever. DEFI (Direct Esterification of Fatty Isethionate) flakes typically contain about 68 to 80 wt. % of sodium fatty acyl isethionate and 15 to 30 wt. % free fatty acid. More than 25 wt. % and no more than 35 % of fatty acyl group of the resulting fatty acyl isethionate have 16 to 18 carbon atoms. Dove^{®} cleansing bar noodles are mixtures of DEFI flakes described above and long chain (mainly C₁₆ and C₁₈) fatty acid and fatty soap which contain about 40 to 55 wt. % of fatty acyl isethionate and 30 to 40 wt. % of fatty acid and fatty soap.

Compositions of the invention may contain non-ionic surfactant. Most preferably non-ionic surfactants are present in the range 0 to 5 wt. %.

Nonionic surfactants that can be included in compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula R-(OCH₂CH₂)ₙOH, where R is an alkyl chain of C12 to C15, and n is 5 to 9.

Other suitable nonionic surfactants include mono- or di-alkyl alkanolamides. Examples include coco mono- or di-ethanolamide and coco monoisopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

RO - (G)n

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

Amphoteric or zwitterionic surfactant can be included in an amount ranging from 0.5 wt. % to about 8 wt. %, preferably from 1 wt. % to 4 wt. % of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

### Silicone Conditioning Agents

The compositions of the invention contain silicone conditioning agents preferably these are emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cSt at 25°C the viscosity of the silicone itself is preferably at least 60,000 cSt, most preferably at least 500,000 cSt, ideally at least 1,000,000 cSt. Preferably the viscosity does not exceed 10⁹ cSt for ease of formulation.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

Combination of amino and non amino functional silicones may also be used.

The total amount of silicone is preferably from 0.01 wt. % to 10 wt. % of the total composition more preferably from 0.1 wt. % to 5 wt. %, most preferably 0.5 wt. % to 3 wt. %.

### Cationic Deposition Polymer

Cationic polymers may be present in the composition of the invention for further enhancing deposition performance.

Suitable cationic polymers may be homopolymers which are cationically substituted or may be formed from two or more types of monomers. The weight average (M_{w}) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine-and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 to 5 %, preferably from 0.05 to 1 %, more preferably from 0.08 to 0.5 % by total weight of cationic polymer based on the total weight of the composition.

### Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof.

Straight chain hydrocarbon oils will preferably contain from about 12 to about 30 carbon atoms. Also suitable are polymeric hydrocarbons of alkenyl monomers, such as C₂-C₆ alkenyl monomers.

Specific examples of suitable hydrocarbon oils include:
paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as C₁-C₂₂ carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt. %.

### Further Optional Ingredients

The compositions of the present invention may also contain adjuncts suitable for hair care. Generally such ingredients are included individually at a level of up to 2, preferably up to 1 wt. % of the total composition.

Suitable hair care adjuncts include perfumes, fibre actives, antidandruff agents, amino acids, sugars, preservatives, pH adjusters and ceramides.

Particulates such as coated mica or insoluble anti-dandruff agents (insoluble zinc salts such as zinc pyrithione) are preferred for use with the invention.

Preferably salt is present at levels from 0.1 to 1 wt. % of the total composition to adjust the product viscosity.

Preferably NaOH is present at levels from 0.1 to 1 wt. % of the total composition to adjust the pH of the formulation.

The invention will now be further illustrated by the following, non-limiting Examples. Examples of the invention are illustrated by a number, comparative Examples are illustrated by a letter.

The invention will be illustrated by the following non-limiting Examples.

### Examples

### Example 1

### Associative Monomer Syntheses

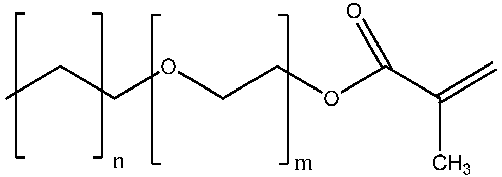

n=12
m=23

Brij® 35P (150g) was dissolved in 500ml anhydrous dichloromethane under a nitrogen atmosphere and cooled in an ice bath to 5°C. Triethylamine (18.6g) was added via syringe before methacryloyl chloride (20.9g) was added dropwise over a 30 minute period. After complete addition, the solution was allowed to warm to room temperature and the reaction stirred for 4 weeks. The solution was then filtered to remove the resulting precipitate and washed once with saturated sodium hydrogen carbonate solution (200ml) and once with saturated brine (200ml). The solution was then passed through a column containing basic alumina before the product was dried with anhydrous magnesium sulphate, filtered and the solvent removed *in vacuo.* In subsequent examples the product is referred to as Surfmer D1.

### HASE copolymer synthesis

A round bottom flask was charged with ethyl acrylate (66.8g), methacrylic acid (37.7g), maleic anhydride (0.515g) and SurmerD1 (10.0g). The mixture was sealed and purged with nitrogen for 60 minutes before sodium dodecyl sulfonate (1.03 g) and deoxygenated water (26.5g) was added and stirred forming a pre-emulsion. A multineck round bottom flask was fitted with a nitrogen sparge and overhead stirrer. Deoxygenated water (181g) and sodium dodecyl sulfonate (0.298g) were added, stirred at 250 rpm and heated to 90°C. Ammonium persulfate (0.073g) in water (1 ml) was added via syringe. The pre-emulsion was fed into the surfactant solution via peristaltic pump over 150 minutes. After complete addition, ammonium persulfate (0.033g) in water (1 ml) was added and the reaction stirred for a further 240 minutes. Copolymers in Table x were synthesised by using suitable adaptations of this process.

**Table 1**

| | **Raw Material Charge (Weight in Grams)** | | | |
|---|---|---|---|---|
| **Component** | Polymer 1.1 | Polymer 1.2 | Polymer 1.3 | Polymer 1.4 |
| **Pre-emulsion** | | | | |
| Ethyl Acrylate | 66.8 | 74.0 | 70.7 | 54.2 |
| Methacrylic Acid | 37.7 | 41.7 | 43.1 | 30.6 |
| Maleic anhydride | 0.515 | 0.57 | 0.71 | 0.506 |
| Surfmer D1 | 10.0 | 4.26 | 7.91 | 8.12 |
| Water | 26.5 | 29.3 | 36.0 | 21.5 |
| Sodium Dodecyl Sulphonate | 1.03 | 1.14 | 1.18 | 0.84 |

| **Surfactant solution** | | | | |
|---|---|---|---|---|
| Deoxygenated Water | 181.0 | 200.6 | 207.3 | 147.1 |
| Sodium Dodecyl Sulphonate | 0.298 | 0.33 | 0.341 | 0.242 |
| Ammonium Persulfate Solution | 0.073 | 0.081 | 0.084 | 0.059 |

| **Post Addition** | | | | |
|---|---|---|---|---|
| Ammonium Persulfate Solution | 0.033 | 0.036 | 0.037 | 0.026 |

**Table 2**

| Polymer composition | MAA w% | MA w% | EA w% | Surfmer w% |
|---|---|---|---|---|
| 1.1 | 32.8 | 0.45 | 58.1 | 8.7 |
| 1.2 | 34.6 | 0.47 | 61.4 | 3.5 |
| 1.3 | 35.2 | 0.58 | 57.7 | 6.5 |
| 1.4 | 32.7 | 0.54 | 58.0 | 8.7 |

The resulting copolymers were used in the Examples below.

### Preparation of Compositions

The polymer was added to water, and then NaOH is added. The polymer swells in water and produces hydrophobic associations. The surfactant was added after swelling. Jaguar was then added to the formulation followed by the remaining ingredients. Finally the pH was adjusted and salt was added to adjust the viscosity.

**Table 3**

| | | **Ex. A** | **Ex. 1** | **Ex.2** | **Ex.3** | **Ex.4** | **Ex.5** | **Ex.6** |
|---|---|---|---|---|---|---|---|---|
| **Chemical name** | **Tradename** | **% W/W** | **% W/W** | **% W/W** | **% W/W** | | | |
| Sodium Laureth Sulfate | Texapon N701 | 12.0 | 12.0 | 12.0 | 12.0 | 12.0 | 12.0 | 12.0 |
| Cocamidopropyl Betaine | Tegobetaine CK | 1.6 | 1.6 | 1.6 | 1.6 | 1.6 | 1.6 | 1.6 |
| Structuring Polymer | Carbopol 980 | 0.6 | - | - | - | | | |
| Structuring Polymer | Example 1.1 Copolymer | - | 0.2 | 0.4 | 0.6 | | | |
| Structuring Polymer | Example 1.2 Copolymer | | | | | 0.4 | | |
| Structuring Polymer | Example 1.3 Copolymer | | | | | | 0.4 | |
| Structuring Polymer | Example 1.4 Copolymer | | | | | | | 0.4 |
| Zinc Pyrithione | Zinc Omadine FPS | 2.1 | 2.1 | 2.1 | 2.1 | 2.1 | 2.1 | 2.1 |
| Zinc Sulfate Heptahydrate | Zinc Sulfate Heptahydrate | 0.1 | 0.1 | 0.1 | 0.1 | 0.1 | 0.1 | 0.1 |
| Dimethiconol/C12-15 Pareth | Silicone Emulsion DC5-7128 | 1.5 | 1.5 | 1.5 | 1.5 | 1.5 | 1.5 | 1.5 |
| Dimethiconol/ TEA-dodecylbenzene sulfonate | Silicone emulsion DC-1788 | 2.6 | 2.6 | 2.6 | 2.6 | 2.6 | 2.6 | 2.6 |
| Guar Hydroxypropyltrimonium Chloride | BFG-Jaguar C17 | 0.2 | 0.2 | 0.2 | 0.2 | 0.2 | 0.2 | 0.2 |
| NaCl | | 0.5 | 0.5 | 0.5 | 0.5 | 0.5 | 0.5 | 0.5 |
| NaOH | | 0.5 | 0.5 | 0.5 | 0.5 | 0.5 | 0.5 | 0.5 |
| Water and minors | Water and minors | q.s. to 100 | q.s. to 100 | q.s. to 100 | q.s. to 100 | q.s. to 100 | q.s. to 100 | q.s. to 100 |
| pH | | 5.5-6.5 | 5.5-6.5 | 5.5-6.5 | 5.5-6.5 | 5.5-6.5 | 5.5-6.5 | 5.5-6.5 |
| **Silicone deposition on virgin hair** | | **1100** | **1210** | **1420** | **1050** | **1500** | **1360** | **1490** |

All products had viscosities adjusted between 2,000 cP and 8,000 cP at 25°C using a Brookfield viscometer at 20 rpm(spindle 5).

### Silicone Deposition Analysis

### Preparation of hair switches for XRD analysis:

2.5 g 6 inch switches of Dark Brown European hair (straight) used.
(1) Base wash
   ∘ 2x base washed with 14 % SLES using 0.1 g/g Sles/Hair = 1.25 g of SLES on 5 switches simultaneously
   ∘ Hair was wet with 37°C 4 L/min water from constant flow tap (10 sec)
   ∘ SLES applied
   ∘ Massaged 30 sec
   ∘ Rinsed 30 sec
   ∘ SLES wash repeated
   ∘ Combed to remove tangles
(2) Shampoo wash
   ∘ Shampoo applied (1.25 g on 5 switches)
   ∘ Massaged 30 sec
   ∘ Rinsed 30 sec
   ∘ Shampoo wash repeated
(3) Drying + cells preparation
   ∘ Left to dry hanging in drying cabinet at 50°C
   ∘ Mounted onto XRF cells and trim excess hair

The examples of the invention had a pearlescent appearance compared with the comparative Example. The examples of the invention deposited silicone to hair to a similar extent to that of the comparative example.

## Claims

1. An aqueous composition comprising:
i) at least 0.5 wt. % of the total composition of a cleansing surfactant
ii) at least 0.01 wt. % of the total composition of a silicone conditioning agent;
iii) at least 0.05 wt. % of the addition polymerization product of:
a. from 0.1 to 5 wt. % of a first unsaturated monomer **A** of a ethylenically unsaturated diacid of formula (I):
HOOC-CR₅=CR₆-COOH (I)
or an unsaturated cyclic anhydride precursor of such an ethylenically unsaturated cis diacid, the anhydride having formula (II) 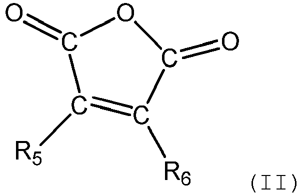 where R₅ and R₆ are individually selected from H, C₁-C₃ alkyl, phenyl, chlorine and bromine;
b. from 1 to 60 wt. % of a second ethylenically unsaturated monomer **B** selected from acrylic acid, methacrylic acid and combinations thereof,
c. from 30 to 75 wt. % a (meth)acrylate monomer **C** selected from C₁ to C₈ alkyl esters of (meth)acrylic acid and C₁ to C₈ alkyl esters of methacrylic acid and combinations thereof,
d. from 1 to 25 wt. % of an associative monomer **D** of the formula III: 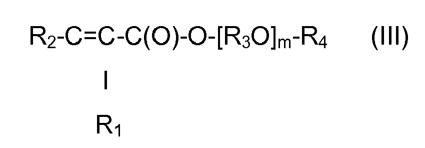
where R₁ and R₂ are each independently selected from H, and C₁₋₃ alkyl; R₃ is C₂-C₄ and mixtures thereof, preferably C₂;
m, the average number of alkoxy units R₃O, is from 6 to 40;
R₄ is alkyl or alkylaryl where the alkyl part is linear or branched; and the total number of carbons is from 6 to 40.
e. from 0 to 1.0 wt. % of a cross linking monomer E for introducing branching and controlling molecular weight, the cross linking monomer comprising polyfunctional units carrying multiple reactive functionalization groups selected from the group consisting of vinyl, allylic and functional mixtures thereof.

2. A composition according to any preceding claim in which the copolymer is at least partially neutralized.

3. A composition according to any preceding claim in which cross linking monomer E is not present.

4. A composition according to any preceding claim in which the monomer A is selected from the group consisting of maleic anhydride, maleic acid and salts thereof.

5. A composition according to any preceding claim in which the monomer B is methacrylic acid.

6. A composition according to any preceding claim in which monomer C of the copolymer is selected from the group consisting of ethylacrylate, methylacrylate, ethylmethacrylate, methylmethacrylate, butylacrylate, butylmethacrylate and mixtures thereof.

7. A composition according to any preceding claim in which monomer C of the copolymer is ethylacrylate.

8. A composition according to any preceding claim in which the monomer E is not present.

9. A composition according to any preceding claim in which associative monomer D is of the formula: 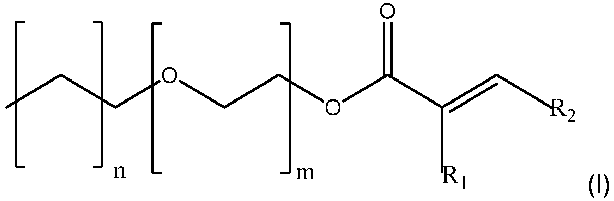
in which each R₁ and R₂ are independently H, C₁ to C₃ alkyl
n ranges from 6 to 40 and m ranges from 6 to 40.

10. A composition according to any preceding claim in which for monomer D n ranges from 12 to 22 and m ranges from 20 to 30.

11. A composition according to any preceding claim in which in monomer D is R₁ is H or methyl and R₂ is H.

12. A composition according to any preceding claim in which for the copolymer:
a ranges from 0.2 to 1 % by weight of the copolymer;
b ranges from 25 to 50 % by weight of the copolymer;
c ranges from 40 to 65 % by weight of the copolymer; and
d ranges from 5 to 15 % by weight of the copolymer
e ranges from 0.01 to 5 wt. % by weight of the copolymer.

13. A composition according to any preceding claim in which the level of copolymer is from 0.05 to 1.0 wt. % of the total composition.

14. A composition according to any preceding claim in which the level of anionic surfactant is from 1.5 wt. % to 20 wt. % of the total composition.

15. A composition according to any preceding claim in which the silicone conditioning agent has an average silicone droplet size of less than 10 micron.

16. A method of treating the hair comprising the step of applying to the hair a composition comprising :
i) at least 0.5 wt. % of the total composition of an anionic surfactant
ii) at least 0.01 wt. % of the total composition of a silicone conditioning agent;
iii) at least 0.05 wt. % of the addition polymerization product of:
a. from 0.1 to 5 wt. % of a first unsaturated monomer **A** of a ethylenically unsaturated diacid of formula (I):
HOOC-CR₅=CR₆-COOH (I)
or an unsaturated cyclic anhydride precursor of such an ethylenically unsaturated cis diacid, the anhydride having formula (II) 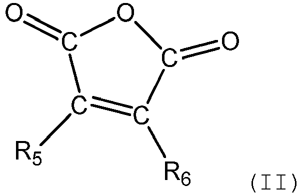 where R₅ and R₆ are individually selected from H, C₁-C₃ alkyl, phenyl, chlorine and bromine;
b. from 1 to 60 wt. % of a second ethylenically unsaturated monomer **B** selected from acrylic acid, methacrylic acid and combinations thereof,
c. from 30 to 75 wt. % a (meth)acrylate monomer **C** selected from C₁ to C₈ alkyl esters of (meth)acrylic acid and C₁ to C₈ alkyl esters of methacrylic acid and combinations thereof,
d. from 1 to 25 wt. % of an associative monomer **D** of the formula III: 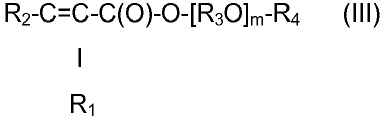
where R₁ and R₂ are each independently selected from H, and C₁₋₃ alkyl; R₃ is C₂-C₄ and mixtures thereof, preferably C₂;
m, the average number of alkoxy units R₃O, is from 6 to 40;
R₄ is alkyl or alkylaryl where the alkyl part is linear or branched; and the total number of carbons is from 6 to 40.
e. from 0 to 1.0 wt. % of a cross linking monomer E for introducing branching and controlling molecular weight, the cross linking monomer comprising polyfunctional units carrying multiple reactive functionalization groups selected from the group consisting of vinyl, allylic and functional mixtures thereof.

## Patentansprüche

1. Wässrige Zusammensetzung, umfassend:
i) mindestens 0,5 Gew.-% der gesamten Zusammensetzung eines Reinigungstensids,
ii) mindestens 0,01 Gew.-% der gesamten Zusammensetzung eines Silikon-Konditioniermittels,
iii) mindestens 0,05 Gew.-% des Additionspolymerisationsproduktes von
a. 0,1 bis 5 Gew.-% eines ersten ungesättigten Monomers **A** einer ethylenisch ungesättigten Disäure der Formel (I):
HOOC-CR₅=CR₆-COOH (I)
oder einer ungesättigten cyclischen Anhydrid-Vorstufe einer solchen ethylenisch ungesättigten cis-Disäure, in welcher das Anhydrid die Formel (II) 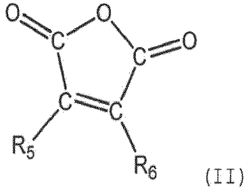 aufweist,
in welcher R₅ und R₆ individuell aus H, C₁-C₃-Alkyl, Phenyl, Chlor und Brom ausgewählt sind,
b. 1 bis 60 Gew.-% eines zweiten ethylenisch ungesättigten Monomers **B**, ausgewählt aus Acrylsäure, Methacrylsäure und Kombinationen davon,
c. 30 bis 75 Gew.-% eines (Meth)acrylat-Monomers **C,** ausgewählt aus C₁-bis C₈-Alkylestern von (Meth)acrylsäure und C₁- bis C₈-Alkylestern von Methacrylsäure und Kombinationen davon,
d. 1 bis 25 Gew.-% eines assoziativen Monomers **D** der Formel III: 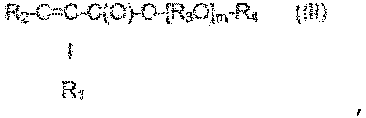
in welcher R₁ und R₂ jeweils unabhängig voneinander aus H und C₁₋₃-Alkyl ausgewählt sind, R₃ C₂-C₄ und Mischungen davon, vorzugsweise C₂, ist,
m, die Durchschnittszahl der Alkoxy-Einheiten R₃O, 6 bis 40 ist,
R₄ Alkyl oder Alkylaryl ist, in welcher der Alkylteil linear oder verzweigt und die gesamte Zahl der Kohlenstoffatome 6 bis 40 ist,
e. 0 bis 1,0 Gew.-% eines vernetzenden Monomers **E** zur Einführung von Verzweigung und zur Steuerung des Molekulargewichts, wobei das vernetzende Monomer polyfunktionelle Einheiten umfasst, die mehrfachreaktive Funktionalisationsgruppen tragen, die aus der aus Vinyl, Allyl und funktionellen Mischungen davon bestehenden Gruppe ausgewählt ist.

2. Zusammensetzung nach irgendeinem vorhergehenden Anspruch, in welcher das Copolymer zumindest partiell neutralisiert ist.

3. Zusammensetzung nach irgendeinem vorhergehenden Anspruch, in welcher das vernetzende Monomer **E** nicht vorliegt.

4. Zusammensetzung nach irgendeinem vorhergehenden Anspruch, in welcher das Monomer **A** aus der aus Maleinsäureanhydrid, Maleinsäure und Salzen davon bestehenden Gruppe ausgewählt ist.

5. Zusammensetzung nach irgendeinem vorhergehenden Anspruch, in welcher das Monomer **B** Methacrylsäure ist.

6. Zusammensetzung nach irgendeinem vorhergehenden Anspruch, in welcher Monomer **C** des Copolymers aus der aus Ethylacrylat, Methacrylat, Ethylmethacrylat, Methylmethacrylat, Butylacrylat, Butylmethacrylat und Mischungen davon bestehenden Gruppe ausgewählt ist.

7. Zusammensetzung nach irgendeinem vorhergehenden Anspruch, in welcher Monomer C des Copolymers Ethylacrylat ist.

8. Zusammensetzung nach irgendeinem vorhergehenden Anspruch, in welcher das Monomer **E** nicht vorliegt.

9. Zusammensetzung nach irgendeinem vorhergehenden Anspruch, in welcher assoziatives Monomer **D** die Formel 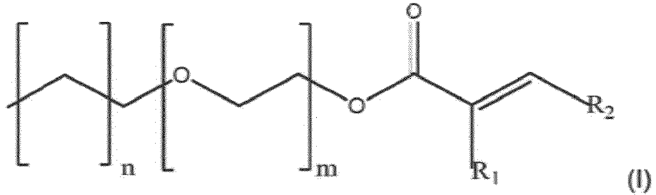 aufweist,
in welcher jedes R₁ und R₂ unabhängig voneinander H, C₁- bis C₃-Alkyl sind,
n zwischen 6 und 40 und m zwischen 6 und 40 liegt.

10. Zusammensetzung nach irgendeinem vorhergehenden Anspruch, in welcher für das Monomer **D** n zwischen 12 und 22 und m zwischen 20 und 30 liegt.

11. Zusammensetzung nach irgendeinem vorhergehenden Anspruch, in welcher im Monomer D R₁ H oder Methyl und R₂ H ist.

12. Zusammensetzung nach irgendeinem vorhergehenden Anspruch, in welcher für das Copolymer
a sich erstreckt von 0,2 bis 1 Gewichts-% des Copolymers,
b sich erstreckt von 25 bis 50 Gewichts-% des Copolymers,
c sich erstreckt von 40 bis 65 Gewichts-% des Copolymers und
d sich erstreckt von 5 bis 15 Gewichts-% des Copolymers,
e sich erstreckt von 0,01 bis 5 Gewichts-% des Copolymers.

13. Zusammensetzung nach irgendeinem vorhergehenden Anspruch, in welcher der Anteil des Copolymers zwischen 0,05 und 1,0 Gew.-% der gesamten Zusammensetzung liegt.

14. Zusammensetzung nach irgendeinem vorhergehenden Anspruch, in welcher der Anteil des anionischen Tensids zwischen 1,5 Gew.-% und 20 Gew.-% der gesamten Zusammensetzung liegt.

15. Zusammensetzung nach irgendeinem vorhergehenden Anspruch, in welcher das Silikon-Konditioniermittel eine durchschnittliche Silikontröpfchengröße von weniger als 10 Mikron aufweist.

16. Verfahren zur Behandlung von Haar, umfassend den Schritt des Auftrags einer Zusammensetzung auf das Haar, umfassend:
i) mindestens 0,5 Gew.-% der gesamten Zusammensetzung eines anionischen Tensids,
ii) mindestens 0,01 Gew.-% der gesamten Zusammensetzung eines Silikon-Konditioniermittels,
iii) mindestens 0,05 Gew.-% des Additionspolymerisationsproduktes von
a. 0,1 bis 5 Gew.-% eines ersten ungesättigten Monomers **A** einer ethylenisch ungesättigten Disäure der Formel (I):
HOOC-CR₅=CR₆-COOH (I)
oder einer ungesättigten cyclischen Anhydrid-Vorstufe einer solchen ethylenisch ungesättigten cis-Disäure, in welcher das Anhydrid die Formel (II) 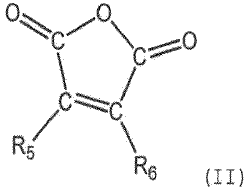 aufweist,
in welcher R₅ und R₆ individuell aus H, C₁-C₃-Alkyl, Phenyl, Chlor und Brom ausgewählt sind,
b. 1 bis 60 Gew.-% eines zweiten ethylenisch ungesättigten Monomers **B,** ausgewählt aus Acrylsäure, Methacrylsäure und Kombinationen davon,
c. 30 bis 75 Gew.-% eines (Meth)acrylat-Monomers **C,** ausgewählt aus C₁-bis C₈-Alkylestern von (Meth)acrylsäure und C₁- bis C₈-Alkylestern von Methacrylsäure und Kombinationen davon,
d. 1 bis 25 Gew.-% eines assoziativen Monomers **D** der Formel III: 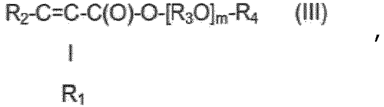
in welcher R₁ und R₂ jeweils unabhängig voneinander aus H und C₁₋₃-Alkyl ausgewählt sind, R₃ C₂-C₄ und Mischungen davon, vorzugsweise C₂, ist,
m, die Durchschnittszahl der Alkoxy-Einheiten R₃O, 6 bis 40 ist,
R₄ ein Alkyl oder Alkylaryl ist, in welcher der Alkylteil linear oder verzweigt und die gesamte Zahl der Kohlenstoffatome 6 bis 40 ist,
e. 0 bis 1,0 Gew.-% eines vernetzenden Monomers **E** zur Einführung von Verzweigung und zur Steuerung des Molekulargewichts, wobei das vernetzende Monomer polyfunktionelle Einheiten umfasst, die mehrfachreaktive Funktionalisationsgruppen tragen, die aus der aus Vinyl, Allyl und funktionellen Mischungen davon bestehenden Gruppe ausgewählt ist.

## Revendications

1. Composition aqueuse comprenant :
i) au moins 0.5 % en masse de la composition totale d'un tensioactif nettoyant
ii) au moins 0.01 % en masse de la composition totale d'un agent de conditionnement de silicone ;
iii) au moins 0.05 % en masse du produit de polymérisation d'addition de :
a. de 0.1 à 5 % en masse d'un premier monomère insaturé A d'un diacide éthyléniquement insaturé de formule (I) :
HOOC-CR₅=CR₆-COOH (I)
ou d'un précurseur d'anhydride cyclique insaturé d'un tel diacide cis éthyléniquement insaturé, l'anhydride présentant la formule (II) 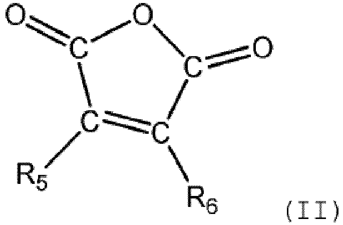 où R₅ et R₆ sont individuellement choisis parmi H, un groupe alkyle en C₁-C₃, phényle, le chlore et le brome ;
b. de 1 à 60 % en masse d'un second monomère éthyléniquement insaturé B choisi parmi l'acide acrylique, l'acide méthacrylique et des combinaisons de ceux-ci,
c. de 30 à 75 % en masse d'un monomère de (méth)acrylate C choisi parmi des esters alkyliques en C₁ à C₈ d'acide (méth)acrylique et des esters alkyliques en C₁ à C₈ d'acide méthacrylique et des combinaisons de ceux-ci,
d. de 1 à 25 % en masse d'un monomère associatif D de la formule III : 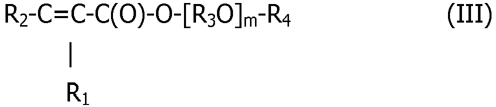
où R₁ et R₂ sont chacun indépendamment choisis parmi H, et un groupe alkyle en C₁₋₃; R₃ est en C₂-C₄ et des mélanges de ceux-ci, de préférence en C₂ ;
m, le nombre moyen d'unités alcoxy R₃O, est de 6 à 40 ;
R₄ est un groupe alkyle ou alkylaryle où la partie alkyle est linéaire ou ramifiée ; et le nombre total d'atomes de carbone est de 6 à 40.
e. de 0 à 1.0 % en masse d'un monomère de réticulation E pour introduire une ramification et contrôler la masse moléculaire, le monomère de réticulation comprenant des unités polyfonctionnelles portant de multiples groupes de fonctionnalisation réactifs choisis dans le groupe constitué d'un groupe vinyle, allylique et de mélanges fonctionnels de ceux-ci.

2. Composition selon l'une quelconque des revendications précédentes, dans laquelle le copolymère est au moins partiellement neutralisé.

3. Composition selon l'une quelconque des revendications précédentes, dans laquelle le monomère de réticulation E n'est pas présent.

4. Composition selon l'une quelconque des revendications précédentes, dans laquelle le monomère A est choisi dans le groupe constitué d'anhydride maléique, d'acide maléique et de sels de ceux-ci.

5. Composition selon l'une quelconque des revendications précédentes, dans laquelle le monomère B est l'acide méthacrylique.

6. Composition selon l'une quelconque des revendications précédentes, dans laquelle le monomère C du copolymère est choisi dans le groupe constitué d'acrylate d'éthyle, d'acrylate de méthyle, de méthacrylate d'éthyle, de méthacrylate de méthyle, d'acrylate de butyle, de méthacrylate de butyle et de mélanges de ceux-ci.

7. Composition selon l'une quelconque des revendications précédentes, dans laquelle le monomère C du copolymère est l'acrylate d'éthyle.

8. Composition selon l'une quelconque des revendications précédentes, dans laquelle le monomère E n'est pas présent.

9. Composition selon l'une quelconque des revendications précédentes, dans laquelle le monomère associatif D est de la formule : 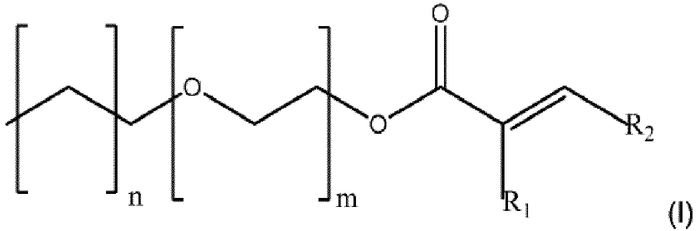
dans laquelle R₁ et R₂ sont chacun indépendamment H, un groupe alkyle en C₁ à C₃.
n est compris entre 6 et 40 et m est compris entre 6 et 40.

10. Composition selon l'une quelconque des revendications précédentes, dans laquelle pour le monomère D n est compris entre 12 et 22 et m est compris entre 20 et 30.

11. Composition selon l'une quelconque des revendications précédentes, dans laquelle dans le monomère D R₁ est H ou le groupe méthyle et R₂ est H.

12. Composition selon l'une quelconque des revendications précédentes, dans laquelle pour le copolymère :
a est de 0.2 à 1 % en masse du copolymère ;
b est de 25 à 50 % en masse du copolymère ;
c est de 40 à 65 % en masse du copolymère ; et
d est de 5 à 15 % en masse du copolymère
e est de 0.01 à 5 % en masse du copolymère.

13. Composition selon l'une quelconque des revendications précédentes, dans laquelle la teneur en copolymère est de 0.05 à 1.0 % en masse de la composition totale.

14. Composition selon l'une quelconque des revendications précédentes, dans laquelle la teneur en tensioactif anionique est de 1.5 % en masse à 20 % en masse de la composition totale.

15. Composition selon l'une quelconque des revendications précédentes, dans laquelle l'agent de conditionnement de silicone présente une taille moyenne de gouttelette de silicone inférieure à 10 microns.

16. Procédé de traitement des cheveux comprenant l'étape d'application aux cheveux d'une composition comprenant :
i) au moins 0.5 % en masse de la composition totale d'un tensioactif anionique
ii) au moins 0.01 % en masse de la composition totale d'un agent conditionnement de silicone ;
iii) au moins 0.05 % en masse du produit de polymérisation d'addition de :
a. de 0.1 à 5 % en masse d'un premier monomère insaturé A d'un diacide éthyléniquement insaturé de formule (I) :
HOOC-CR₅=CR₆-COOH (I)
ou d'un précurseur d'anhydride cyclique insaturé d'un tel diacide cis éthyléniquement insaturé, l'anhydride présentant la formule (II) 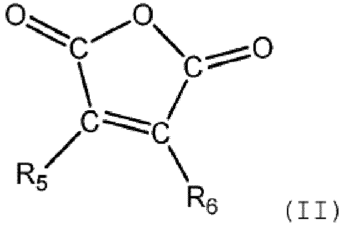 où R₅ et R₆ sont individuellement choisis parmi H, un groupe alkyle en C₁-C₃, phényle, le chlore et le brome;
b. de 1 à 60 % en masse d'un second monomère éthyléniquement insaturé B choisi parmi l'acide acrylique, l'acide méthacrylique et des combinaisons de ceux-ci,
c. de 30 à 75 % en masse d'un monomère de (méth)acrylate C choisi parmi des esters alkyliques en C₁ à C₈ d'acide (méth)acrylique et des esters alkyliques en C₁ à C₈ d'acide méthacrylique et des combinaisons de ceux-ci,
d. de 1 à 25 % en masse d'un monomère associatif D de la formule III : 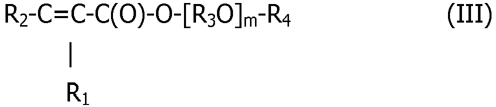
où R₁ et R₂ sont chacun indépendamment choisis parmi H, et un groupe alkyle en C₁₋₃ ; R₃ est en C₂-C₄ et des mélanges de ceux-ci, de préférence en C₂ ;
m, le nombre moyen d'unités alcoxy R₃O, est de 6 à 40 ;
R₄ est un groupe alkyle ou alkylaryle où la partie alkyle est linéaire ou ramifiée ; et le nombre total d'atomes de carbone est de 6 à 40.
e. de 0 à 1.0 % en masse d'un monomère de réticulation E pour introduire une ramification et contrôler la masse moléculaire, le monomère de réticulation comprenant des unités polyfonctionnelles portant de multiples groupes de fonctionnalisation réactifs choisis dans le groupe constitué d'un groupe vinyle, allylique et des mélanges fonctionnels de ceux-ci.
